# EUROPEAN PATENT APPLICATION

(11) **EP 1 609 485 A1**
(43) Date of publication of application: **28.12.2005**
(21) Application number: 04014699.5
(22) Date of filing: 23.06.2004
(51) Int. Cl.: A61L 2/10, A61L 2/08

(54) **Method for the inactivation of viral components of protein-containing biological compositions**

(71) Applicant: ZLB Behring GmbH, 35041 Marburg (DE)
(72) Inventor: Lengsfeld, Thomas, Dr., 35041 Marburg (DE); Grandgeorge, Michel, Dr., 69670 Vaugneray (FR); Schäfer, Wolfram, Dr., 35117 Münchhausen (DE); Nowak, Thomas, Dr., 35460 Staufenberg (DE); Gröner, Albrecht, Dr., 64342 Seeheim-Jugenheim (DE)
(74) Representative: Pfeil, Hugo

(57) **Abstract**

The present invention relates to a method for inactivating microorganisms, especially viruses, in protein containing biological compositions, especially solutions of therapeutic proteins extracted from blood, serum, plasma or other body fluids like clotting factors, immunoglobulins, albumins, haemoglobin, or solutions of proteins obtained by extraction of other biological materials or by biotechnological techniques. The method also applies to the inactivation of microorganisms in protein containing preparations which are intended for use in inactivated or non-living vaccines.

## Description

The present invention relates to a method for inactivating microorganisms, especially viruses, in protein containing biological compositions, especially solutions of therapeutic proteins extracted from blood, serum, plasma or other body fluids like clotting factors, immunoglobulins, albumins, haemoglobin, or solutions of proteins obtained by extraction of other biological materials or by biotechnological techniques. The method also applies to the inactivation of microorganisms in protein containing preparations which are intended for use in inactivated or non-living vaccines.

The present invention relates also to the blood or plasma component or derivative or the protein containing composition treated by the method of the present invention as well as to the pharmaceuticals or veterinary medicines comprising the said proteins.

It is a constant concern in the manufacture of pharmaceuticals to assure that no microbial contaminants can be transmitted to the recipient or user of such products. However, the protein containing pharmaceuticals are generally sourced from biological materials which may contain viruses or their manufacturing process makes use of reagents or a culture medium which may themselves come from a biological source and therefore may be contaminated by viruses. International guidelines, e.g. ICH Topic Q5A, therefore, recommend to introduce in the manufacturing processes of protein based biopharmaceuticals dedicated steps to inactivate or remove those potential viral contaminants. The European Note for Guidance CPMP/BWP/268/95 of 14 February 1996 defines what is a clearly effective method for inactivating or removing viruses as a method which is able to reduce infectivity by about 4 Log (= 4 log₁₀) or more.

An inherent difficulty in the task to inactivate or remove viruses in/from protein containing biological composition is due to the extreme diversity of viruses, different by their size, their structural composition as having or not an envelope (which may, in addition, differ in the relative amount of lipids), their genome which may consist of single or double stranded RNA or DNA. These and other differences explain that many known inactivating methods work well on some virus categories but not on others. For example the well-known solvent-detergent method does not work on non-enveloped viruses. Also, among non-enveloped viruses some can be easily inactivated by heat or by acid pH treatment, others are resistant to heat inactivation, e.g. parvoviruses, or to acid pH inactivation, e.g. polioviruses.

On the other hand, therapeutic proteins are in general of complex structure and quite fragile, i.e. sensitive to degradation (modification of their primary structure) and/or denaturation (modification of their secondary, tertiary and quaternary structures), which make them difficult to withstand aggressive virus inactivating methods. These molecular modifications may result in a loss of their biological activity or antigenic properties, in a reduced stability in their pharmaceutical form upon storage, and in new immunogenicity properties, which might put the recipient of such products at risk of allergic reactions upon repeated administration or application.

There is, therefore, a need of having a clearly effective virus inactivation method which would work in all categories of viruses including novel and unpredictable virus contaminants, and which would be compatible with the manufacturing of therapeutic proteins.

Light inactivation is a technology, which consists in irradiating a biological composition, in a liquid or dried form using a source of IR and/or visible light and/or UV light or x- or γ-rays. In the case of low energy light, e.g. visible or UVA light, it is necessary to use the light in combination with photosensitizers like methylene blue, psoralens or riboflavin. Light inactivation occurs either by direct of indirect (through sensitizers) transfer of photon energy to the targeted biomolecule. It may also involve oxidative mechanisms due to the formation of reactive oxygen species (ROS), which may form by activation of oxygen or water during the irradiation process.

In principle light inactivation is able to preferentially target nucleic acids rather than proteins, which makes this technology attractive in the field of therapeutic proteins. This is possible because of the nucleic acid intercalating or affinity property of the photosensitizer used, or because of the preferential absorption of UVC, e.g. at a wavelength of 254 nm, by nucleic acid components in contrast to proteins, and because of the greater sensitivity of nucleic acids to light degradation due to their larger size than proteins and use of the fact that a single degradation point in their nucleotide sequence may theoretically prevent their future correct replication and therefore the multiplication of the corresponding virus.

Light inactivation technology has been tried and sometimes used since the mid 50s of last century especially in the field of blood and plasma derivatives but with mixed success until recently. One major reason of this low success is that at the energy necessary to inactivate the most resistant viruses, e.g. those, which have double-stranded DNA allowing repair mechanisms in an inoculated cell, significant damage (mainly oxidative) also occurs to the protein(s) of interest.

Therefore, although equipment of machines now exist which would make the light inactivation technology validatable and transferable to modern transfusion institutions or pharmaceutical plants under full GMP conditions, e.g. the static mixer equipped in-flow irradiator described in GB 2 200 020, this technology is not currently used in the manufacturing of any licensed blood product or pharmaceutical, except for some particularly robust products like a crude human plasma fraction or UVC or gamma irradiated animal serum fractions, animal peptones or some enzymes used as culture medium ingredients or as reagents in the manufacture of some cell culture derived biotechnology medicinal products or vaccines.

Some proposals have been made in order to limit the damage of proteins during light inactivation while achieving a clearly effective virus inactivation, i.e. about our more than 4 Log titer reduction:
In US patent 6,190,608 B1 and US Patent Application No. 2001/0046450 A1 it is proposed to use a UVC tubular flow cell irradiator to inactivate non-enveloped viruses in plasma derivates, whereby adjusting the energy applied to below 640 J/m² but at a sufficient level to retain more than 85% of the plasma derivative activity. According to the presented data the method works well on single-stranded virus EMC (encephalomyocarditis virus) and MVM (murine virus), but resulted in a considerably lower virus reduction factor for the enveloped double-stranded virus BHV (2.5 to 3.0 Log reduction only in various plasma derivates).
In US patent 5,981,163 it is proposed to protect proteins against oxidative damage during irradiation using a combination of ROS quenchers to type I (quenchers of free radical ROS species) and ROS quenchers type II (quenchers of the ROS molecule singlet oxygen), or to use molecules which quench both ROS types. As typical examples of quenchers type I or type II the following compounds are claimed: mannitol, glycerol, glutathione, SOD (superoxide dismutase) (all type I quenchers), histidine, tryptophane (both type II quenchers) and ascorbate (not indicated if a type I or type II). As examples of quenchers which quench both type I and type II only flavonoid compounds are claimed, in particular rutin. Rutin is given as the most effective protectant of all, more effective than a combination of type I and type II quenchers. The inventors confirmed the superiority of rutin in several publications where they report the use of UVC inactivation on plasma and various plasma derivatives (Factor VIII, albumin, immunoglobulins and fibrinogen; see for instance Photochemistry and Photobiology 1997, 65(3):432-435). However, the authors of the 1997 paper report a reduction of the inactivation of EMC virus in albumin in presence of 1.6 mmol/L rutin but no significant protection at this concentration of rutin against albumin degradation/denaturation as evidenced by the formation of albumin dimers and larger aggregates. The removal of these aggregates had to be performed in a subsequent purification step by filtration through a Sephacryl S200 HR column. Furthermore rutin, a complex molecule of a high molecular weight (610 Da), is poorly soluble in aqueous solutions and therefore difficult to remove from the product after the inactivation step. In addition, there is some indication that rutin has mutagenic properties, which could be a serious concern for use in the production of medicinal products ( Mutation Research 1986, 170:103-113).

Glutathione (γ-glutamylcysteinylglycine) has been cited as a possible protein protectant against oxidation, or ROS quencher, during light inactivation, for example in the above-mentioned US patent 5,981,163. In this patent glutathione was taken as a model for thiol compounds and listed as a type I quencher. However, glutathione was not found to be effective in protecting the target proteins during light inactivation. A similar conclusion was drawn for all other type I quenchers assayed as well as for all type II quenchers, which led the inventors to propose the combination of a type I quencher and a type II quencher, as the only effective quencher formulation. In-vivo, glutathione plays a central role in the physiological defence and defence regulation against oxidative stress in cellular systems. This is mainly due to the fact that inside living organism the redox environment is dependent from the availability of reduced glutathione versus oxidized glutathione. On average the reduced glutathione concentration in the cytosol is 1 to 10 mmol/L. In-vitro, outside living organisms, free thiols are however not under control of reductases and other systems responsible for maintaining an optimal redox environment and therefore isolated proteins are often structurally affected by free thiols, which can reduce and disrupt intra-domain or inter-domain/inter-chain disulfide bonds, especially under stress conditions caused by heat, irradiation or chaotropic agents.

Other commonly described low molecular weight thiolsinclude for example cysteine, the major low molecular thiol in human plasma (about 0.01 to 0.02 mmol/L plasma), homocysteine and cysteinylglycine, also present in human plasma, N-acetylcysteine a natural metabolite of cysteine and the synthetic chemical reagents β-mercaptoethanol, dithiothreitol and dithioerythritol. Cysteine, β-mercaptoethanol and dithiothreitol are more reactive than the relatively larger and more acidic molecule glutathione. β-mercaptoethanol and dithiothreitol are common chemical reagents used in various biochemical processes or analytics with the purpose to quantitatively reduce/disrupt the disulfide bridges of proteins, e.g. in the manufacture of reduced/alkylated immunoglobulins, in the solubilization of misfolded insoluble recombinant protein from E.coli or in the preparation of protein samples for analysis with the sodium dodecylsufalte-polyacrylamide gel electrophoretic method (SDS-PAGE, reducing conditions).

From European patent application 1 145 669, a method for sterilizing a protein containing biological composition is known comprising the step of subjecting said composition to a virucidally effective amount of artificial irradiation in the presence of a phenol aldehyde derivative like vanillin, which protects the protein during irradiation against decomposition. Vanillin, however, is not a physiological substance in humans and absorbs light at a wavelength of 254 nm.

The present invention aims to obtain a new sterilization method for inactivating microorganisms especially viruses of all kind, enveloped and non-enveloped viruses, to a significant degree in protein containing compositions while minimizing the damage to the protein(s) of interest, and without the disadvantages inherent to other methods.

Another aim is to improve the safety of protein containing pharmaceuticals for human or animal use, by including the new sterilization method in their manufacturing process potentially in combination with other methods for virus inactivation and/or removal like heat treatment (protein in aqueous stabilized solution or lyophilized), treatment with solvent-detergent, precipitation by ammonium sulphate or polyethyleneglycol, chromatographic purification, or nanofiltration.

It has now been found that this aim is reached by a method of inactivation of viral components in a protein containing biological composition comprising the step of subjecting said composition to a virucidally effective amount of irradiation in the presence of a low molecular mono-thiol compound which does not, or only to a very small extend, absorb light in the range of about 250 - 260 nm and is used in a concentration which retains at least 80%, preferably at least 90%, most preferably at least 95% % of the biological activity of the protein after treatment with irradiation.

Without being able to provide a sound scientific rationale we found that the most effective substances for use in this invention are low-molecular weight, i.e. substances with a molecular weight of less than 200 Da, mono-thiols.

By such a method the light energy applied on the protein containing biological composition is sufficient to obtain an effective virus kill whereby the protein is protected from damage caused by the irradiation process by the low molecular weight thiol compound under conditions such that no substantial disulfide bond disruptive effect of the low molecular weight thiol on the protein is detectable. These conditions consist in a careful adjustment of the thiol concentration. It was found that cysteine performed especially well, better than the di-thiol compound dithiothreitol.

Good results have been obtained when a mono-thiol compound from the group of cysteine, homocysteine, N-acetylcysteine, cysteinylglycine and β-mercaptoethanol was used in an amount of 0.1 to about 4 mmol/L.

Cysteine is a natural component of proteins and a constituent of intra- and intermolecular protein disulfide bridges. Cysteine is the only low molecular weight thiol approved for food use (Sapers et al. on p. 551 in: Food Additives; ed. A.L. Branen et al. 2002, Marcel Dekker, Inc.), and is classified as GRAS (Generally Regarded as Safe).

Low molecular weight thiols were compared to rutin and other stabilizers in various experiments using a UVC in-flow irradiator similar to the one described in GB 2 200 020 and a fibrinogen containing compositions as well as a factor VIII containing composition as models for fragile proteins. Degradation/ denaturation of these proteins caused by UVC irradiation was evaluated by measuring their biological activity or their aggregates content before and after irradiation. Virus inactivation was evaluated in spiking experiments with various model viruses especially a parvovirus - CPV = canine parvovirus (a non-enveloped virus with a single-stranded DNA), a picornavirus - polio-1 = poliomyelitis virus type 1 (a non-enveloped virus with a single-stranded RNA) and a herpes virus - PRV = pseudorabies virus (an enveloped virus with a double-stranded DNA). In these experiments, cysteine compared better to rutin or other stabilizers, allowing a higher virus reduction at equal irradiation doses. Moreover, in the presence of cysteine at a concentration sufficient to protect proteins, equal virus inactivation factors where achieved when compared to irradiation of the non-stabilized solution. Unlike other stabilizers such as rutin and ascorbic acid, cysteine like the other low molecular thiol cited in this document does not adsorb UVC light at 254 nm and thus does not reduce the effective UVC irradiation dose acting on the genome of the virus or microorganism. In addition, cysteine has not the drawback of rutin and many other stabilisers in terms of structure complexity, low water solubility, and concerns regarding mutagenicity.

In establishing the optimal range of the concentration of the thiol compound the disulfide bond reducing/ disrupting effect of cysteine was monitored by SDS-PAGE (non-reducing conditions). In the case of fibrinogen, disulfide bond disruption translates in the liberation of the alpha, beta and gamma chains of fibrinogen, which appear on the gels as extra low molecular weight bands. This range should be established similarly for every low molecular weight thiol and every protein. However, fibrinogen alpha, beta and gamma chain liberation can be considered as a good and simple screening model to assess disulfide bond disruption risk for every protein. Some type I or type II quenchers suggested in the US patent 5,981,163 as possible stabilizers where also tested alone or in combination (see examples of this application) without any success. Cysteine shows here exceptional properties in protecting proteins during an irradiation process which is not shown by known type I and type II substances, either alone or in combination.

The method of the invention provides proteins which retain at least 80%, preferably at least 90%, most preferably at least 95% of their biological activity after treatment with irradiation. At the same time a disulfide bond reduction of not more than 1% is observed. The amount of protein aggregates formed during irradiations remains below 5%, preferably below 3%, most preferably below 1%.

This method is especially suitable for protein containing biological compositions comprising purified plasma proteins, especially coagulation factors, like factor II, V, VII, VIII, IX, X, XI, XII, fibrinogen and the von Willebrand factor either alone or in combination with factor VIII.

Especially favourable results are obtained if said protein containing biological composition is subjected so said irradiation in the presence of cysteine, homocysteine, N-acetyl-cysteine, cysteinylglycine or β-mercaptoethanol and if either before, after or at the same time of such treatment the composition is subjected additionally to at least one different virucidal method. Such additional treatment may be any of the methods known to the one skilled in the art, e.g. heat treatment, pH manipulation, solvent-detergent treatment, precipitation by ammonium sulphate or polyethyleneglycol, chromatographic purification, nanofiltration or irradiation treatment.

The biological composition obtained by the treatment in accordance with the invention may be screened in order to find out the degree of disulfide bond reduction. Thus in a screening assay using fibrinogen and SDS-PAGE (non-reducing) with coomassie blue staining the generation of free alpha-, beta- or gamma chains from fibrinogen is indicated. It may be also possible to use fibrinogen in the SDS-PAGE assay as a universal indicator of disulfide bond reductive potential of a given thiol substance.

The following examples will describe in more details the conditions and results of all these experiments.

### Examples

### Example 1

Fibrinogen (5 g/L fibrinogen, buffered in 50 mmol/L NaCl, 20 mmol/L Na Citrate, pH 7.3) non-stabilized or stabilized with cysteine or rutin was treated with a UVC in-flow irradiator equipped with a static mixer as described in GB 2 200 020.

The non-stabilized and stabilized solutions were spiked with polio-1 for a final concentration of 7.3 log₁₀ CCID₅₀/mL.
The UVC illuminated tube of the irradiator had a 6 mm diameter and a length of 35 cm. The illuminated tube was surrounded by four UVC lamps (15 W each, 40 cm length, Philips TUV 15W, Netherlands) providing a maximal emission at 254 nm.

The materials were pumped using a peristaltic pump with a flow rate of 25 mL/min and re-circulated several times through the irradiator in order to achieve various irradiation time expressed in seconds.

Protein aggregate formation as a measure for protein denaturation was studied by SE (Size Exclusion)-HPLC using TSK G 4000SWXL column, TosoHaas, Japan in parallel experiments without virus spike. The results were expressed as increase in fibrinogen aggregates. 1 % increase in fibrinogen aggregates means that the percentage of aggregates in the solution has increased by 1 point (for example from 10% to 11 %).

Non-stabilized solutions and solutions stabilized with 1 mmol/L cysteine or 5 mmol/L cysteine showed comparable inactivation of polio-1 while in the presence of 0.5 mmol/L rutin inactivation of polio-1 was significantly reduced. After 5.8 s irradiation there was an increase in fibrinogen aggregates of 10% in the non-stabilized solution while the aggregate increase was below 1% in the presence of 0.1 mmol/L rutin, 1 mmol/L cysteine and 5 mmol/L cysteine (see Table 1).

Conclusion: Cysteine was found to be superior to rutin with regard to virus inactivation by UVC at identical irradiation dose. A possible explanation for this effect might be the light absorption by rutin (optical density at 254 nm = OD₂₅₄ₙₘ, see Table 1).

**Table 1:**

| Inactivation of polio-1 in stabilized or non-stabilized fibrinogen solution after 5.8 s UVC irradiation time | | | |
|---|---|---|---|
| **Stabiliser** | **Virus Reduction Factor [log**_{**10**}**]** | **Increase in fibrinogen aggregates [%]** | **OD**_{**254nm**} **of fibrinogen solution** |
| Non-stabilized solution | 5.4 | 10 | 3.3 |
| 0.1 mmol/L rutin | 3.9 | <1 | 6 |
| 1 mmol/L cysteine | 5.5 | <1 | 3.3 |
| 5 mmol/L cysteine | 5.0 | <1 | 3.3 |

### Example 2

Fibrinogen (5g/L fibrinogen, buffered in 50 mmol/L NaCl, 20 mmol/L NaCitrate, pH 7.3) non-stabilized or stabilized with 0.5 mmol/L rutin and 5 mmol/L cysteine was treated with a UVC in-flow irradiator and under conditions as described in example 1 with the exception that the irradiation tube length was 12.5 cm.

The non-stabilized and stabilized solutions were spiked with CPV for a final concentration of 7.0 log₁₀ CCID₅₀/mL.

Non-stabilized solution and solution stabilized with 1 mmol/L cysteine showed comparable inactivation of CPV while in the presence of 0.5 mmol/L rutin inactivation was significantly reduced (See Table 2).

Conclusions: Cysteine was again found to be superior to rutin with regard to virus inactivation by UVC at identical irradiation doses.

**Table 2:**

| Inactivation of CPV in stabilized or non-stabilized fibrinogen solution | | | | |
|---|---|---|---|---|
| Irradiation time [s] | 0.7 | 1.3 | 2.0 | |
| Virus reduction factors [log₁₀] | 2.3 | 4.2 | 6.7 | non-stabilized |
| | 0.9 | 1.6 | 2.6 | 0.5 mM rutin |
| | 2.4 | 4.2 | 6.5 | 1 mM cysteine |

### Example 3

Fibrinogen (5 g/L fibrinogen, buffered in 50 mmol/L NaCl, 20 mmol/L NaCitrate, pH 7.3) non-stabilized or stabilized with 0.5 mmol/L rutin, 2 mmol/L ascorbic acid and 5 mmol/L cysteine was irradiated for 16 s with a UVC in-flow irradiator and under conditions as described in example 1.

The non-stabilized and stabilized solutions were spiked with PRV for a final concentration of 7.0 log₁₀CCID₅₀/mL.

SE-HPLC was performed as in example 1 to measure fibrinogen aggregate formation.

Non-stabilized solution and solution stabilized with 5 mmol/L cysteine showed comparable inactivation of PRV while in the presence of 0.5 mmol/L rutin and 2 mmol/L ascorbic acid inactivation of PRV was significantly reduced. Optical density of the fibrinogen solution at 254 nm (OD₂₅₄ₙₘ) was unaltered by addition of cysteine to 5 mmol/L final concentration while it was increased by addition of rutin to 0.5 mmol/L and of ascorbic acid to 2 mmol/L final concentration (see Table 3).

Conclusion: Rutin and ascorbic acid were inferior to cysteine with regard to PRV inactivation.

**Table 3:**

| Inactivation of PRV in stabilized or non-stabilized fibrinogen solution by irradiation for 16 s | | | |
|---|---|---|---|
| | Virus Reduction Factor [log₁₀] | OD₂₅₄ₙₘ | Increase in fibrinogen aggregates [%] |
| non-stabilized | 4.0 | 3.3 | 3.0 |
| 0.5 mmol/L rutin | 3.0 | 13 | 1 |
| 2 mmol/L ascorbic acid | 2.0 | 20 | 1 |
| 5 mmol/L cysteine | 4.0 | 3.3 | <1 |

### Example 4

Fibrinogen (5 g/ L fibrinogen, buffered in 50 NaCl, 20 mmol/ L NaCitrate, pH 7.3) non-stabilized or stabilized with mannitol (type I quencher according to US 5,981,163) as mentioned in US 5,981,163 at two concentrations (2 mmol/ L; 100 mmol/ L) in two separate experiments was treated with the irradiator and the same irradiation conditions as described in example 1 (irradiation time 12 s). Formulations with histidine (type II quencher according to US 5,981,163) were tested at 2 mmol/L, 5 mmol/ L and in a mixed formulation with mannitol (1 mmol/ L histidine and 100 mmol/ L mannitol).

Protein aggregate formation was measured as described in example 1.

A 20 % increase in aggregates was found after treatment of the non-stabilized solutions. 7 % increase in aggregates was found after treatment of the 2 mmol/ L mannitol stabilized solution. 9 % new aggregates were found after treatment of the 100 mmol/ L mannitol stabilized solution. 10 % new aggregates were found after treatment of the 2 mmol/ L histidine stabilized solution. 12 % new aggregates were found after treatment of the 5 mmol/ L histidine stabilized solution. 9 % new aggregates were found after treatment of the histidine + mannitol stabilized solution (see Table 4).

Conclusion: none of the tested protectants provided an acceptable protection of fibrinogen against aggregation during irradiation

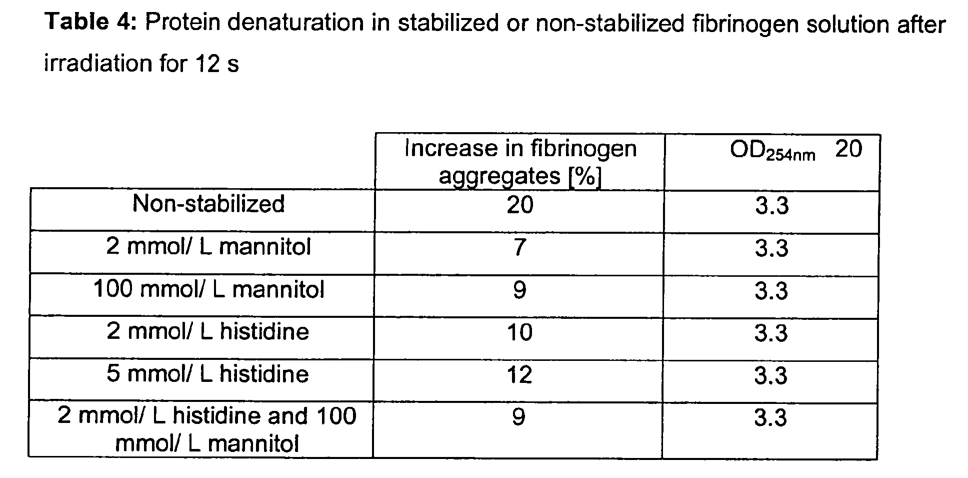

### Example 5

5a) Fibrinogen (5 g/L fibrinogen, buffered in 50 mmol/L NaCl, 20 mmol/L NaCitrate, pH 7.3) was stabilized with various protectants: 2 mmol/L glutathione or 0.5, 1, 2 or 5 mmol/L cysteine or 0.25, 0.5, 1 or 2 mmol/L dithiothreitol and incubated for 1 h and 24 h at room temperature.

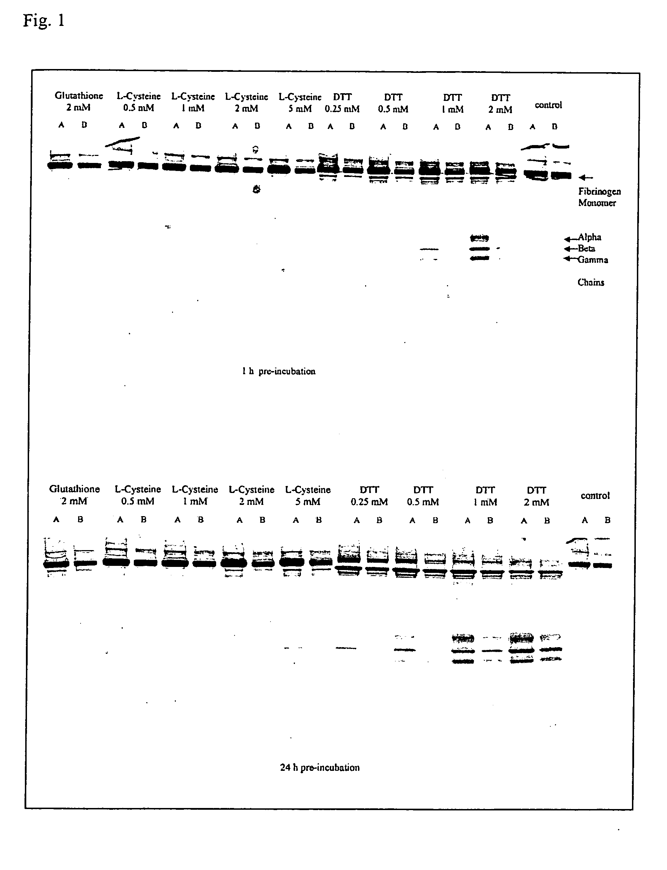

The non-stabilized and stabilized solutions were analysed by SDS-PAGE (non reducing conditions) without heating of the samples in the SDS buffer prior to the electrophoretic separation of the proteins. After 1 h pre-incubation, fibrinogen was found to be partially reduced to its alpha, beta and gamma chains in all samples stabilized with 1 and 2 mmol/L dithiothreitol (faint traces of reduced fibrinogen were also observed with 0.5 mmol/L dithiothreitol). These chains appeared as bands of lower molecular weight than fibrinogen on the gels. After 1 h pre-incubation, no reduced fibrinogen was detected in non-stabilized, 2 mmol/L glutathione, 0.25 mmol/L dithiothreitol and 0.5 to 5 mmol/L cysteine stabilized formulations (see Figure 1). After 24 h pre-incubation, fibrinogen was found to be partially reduced to its alpha, beta and gamma chains in all samples stabilized with 5 mmol/L cysteine and 0.25 to 2 mmol/L dithiothreitol. After 24 h pre-incubation, no reduced fibrinogen was detected in non-stabilized, 2 mmol/L glutathione and 0.5 to 2 mmol/L cysteine stabilized formulations (see Figure 1).

5b) The 0.25 mmol/L dithiothreitol, 2 mmol/L glutathione and 1 mmol/L cysteine stabilized fibrinogen solutions as well as a non-stabilized fibrinogen solution were UVC-treated as described in example 1 (irradiation time 12 s).

Fibrinogen aggregate formation was measured by SE-HPLC as described in example 1. An increase in aggregates of 20% was found after treatment of the non-stabilized solution. No increase in aggregates was found in the 1 mmol/L cysteine formulation, 1.5 % increase in aggregates was found in the 0.25 mmol/L dithiothreitol formulation and 1.5% increase of aggregates was found in the 2 mmol/L glutathione stabilized formulation.

Fibrinogen activity of samples was measured as clottable activity according to Clauss. More than 50% loss in activity was observed in the 0.25 mmol/L DTT stabilized solution before and after irradiation. After irradiation of samples stabilized with 2 mmol/L glutathione a 20% loss of activity was measured. After irradiation of samples stabilized with 1 mmol/L cysteine, only a 10% loss of activity was observed. Due to the high aggregate increase in the samples of non-stabilized fibrinogen, the Clauss method was not applicable (see Table 5).

Conclusion: The protection of fibrinogen against UVC irradiation damage was best with cysteine. The strong reducing effect of dithiothreitol seriously damages fibrinogen beyond what is detected on SDS-PAGE.

**Table 5 :**

| Protein denaturation in stabilized or non-stabilized fibrinogen solution after irradiation for 12 s | | |
|---|---|---|
| **Formulation irradiated** | **% new aggregates** | **% loss of activity** |
| Non-stabilized | 20 | not measurable |
| 0.25 mmol/L dithiothreitol | 1.5 | >50 |
| 1 mmol/L glutathione | 1.5 | 20 |
| 1 mmol/L cysteine | not detectable | 10 |

### Example 6

Fibrinogen (5 g/L fibrinogen, buffered in 50 mmol/L NaCl, 20 mmol/L NaCitrate, pH 7.3) non-stabilized or stabilized with different concentrations of β-mercaptoethanol (0.25; 0.5; 1 and 2 mmol/L) was treated with the irradiator and the same irradiation conditions as described in example 1 (irradiation time 12 s).

A SDS-PAGE analysis was performed after one hour and 24 hours incubation without irradiation as described in example 5a. No reduction of fibrinogen in its alpha, beta and gamma chains was observed in any of the stabilized solutions.

Fibrinogen aggregate formation was measured by SE-HPLC as described in example 1. Less than 1% increase in aggregates was measured in all the stabilized irradiated formulations. In the non-stabilized solution an increase in aggregates of 20% was measured. The fibrinogen activity was measured as in example 5b. A loss of activity of 13% to 17% during irradiation was measured in the β-mercaptoethanol stabilized solutions.

Conclusions: The protection of Fibrinogen against UVC irradiation damage was significant at concentrations of β-mercaptoethanol which was not causing disruption of disulfide bonds and reduction of fibrinogen to its sub-chains alpha, beta and gamma.

### Example 7

Fibrinogen (5 g/I fibrinogen, buffered in 50 mmol/L NaCl, 20 mmol/L NaCitrate, pH 7.3) non-stabilized or stabilized with different concentrations of cysteine (0.01; 0.05; 0.125; 0.25; 0.5; 1 and 2 mmol/L) was treated with the irradiator and the same irradiation conditions as described in example 1 (irradiation time 12 s).

Protein aggregate formation was measured as described in example 1. Results are shown in Table 6.

**Table 6:**

| Fibrinogen aggregate formation after 12 s UVC treatment in the presence of various concentrations of cysteine | |
|---|---|
| **Formulation irradiated** | **% new aggregates** |
| non stabilized | 18 |
| 0,01 mmol/L Cysteine | 11 |
| 0,05 mmol/L Cysteine | 2 |
| 0,125 mmol/L Cysteine | 1,5 |
| 0,25 mmol/L Cysteine | 1 |
| 0,5 mmol/L Cysteine | <1 |
| 1 mmol/L Cysteine | <1 |
| 2 mmol/L Cysteine | <1 |

Conclusion: not more than 1.5% fibrinogen aggregates were formed at cysteine concentration above 0.1 mmol/L. No detectable increase (<1%) of aggregates was detected at 0.5 mmol/L cysteine concentration and above.

### Example 8

Factor VIII (in 5 g/L total protein solution, buffered in Citrate/NaCl/Glycine, containing human albumin, pH 7.3) non-stabilized or stabilized with 0.5 mmol/L cysteine. The non-stabilized and the 0.5 mmol/L cysteine stabilized solutions were spiked with polio-1 to a final concentration of 7.3 log₁₀CCID₅₀mL.

The solutions were treated with the irradiator and under conditions as described in example 1.

The materials were irradiated for 5 s.

FVIII activity was measured by a FVIII chromogenic assay. A 20% decrease in FVIII activity was found in the non-stabilized solution after treatment. Only a 7% decrease in FVIII activity was detected after the treatment in presence of 0.5 mmol/L cysteine.

After irradiation it was found, that polio-1 was inactivated by more than 4 Log both in stabilized and non-stabilized solution.

Conclusion: Cysteine at 0.5 mmol/L protected effectively the Factor VIII from denaturation.

### Legend to the Figure

**Fig. 1:** SDS-PAGE of example 5a: fibrinogen samples were pre-incubated for 1 h and 24 h with and without low molecular weight thiols. 10 µg/20 µL sample (lane A) and 5 µg/20 µL sample (lane B) were separated in a 8 x 8 cm SDS-PAGE system (4 - 20% acrylamide, 1.5 mm gel thickness, tris-glycine buffer). Proteins were detected with coomassie blue stain. DTT= dithiothreitol.

## Claims

1. Method for the inactivation of viral components of a protein-containing biological composition comprising the step of subjecting said composition to a virucidally effective amount of irradiation in the presence of a low molecular mono-thiol compound which does not absorb light in the range of about 250 - 260 nm and is used in a concentration which retains at least 80 % of the biological activity of the protein after the treatment with irradiation.

2. The method as claimed in claim 1, wherein the irradiation is UV or visible light.

3. The method as claimed in claim 2, wherein the UV irradiation is UVA, UVB or UVC.

4. The method as claimed in claim 3, wherein the UVC irradiation has a wavelength of about 254 nm.

5. The method as claimed in claim 1, wherein the low molecular mono-thiol compound is selected from the group consisting of cysteine, homocysteine, N-acetyl-cysteine, cysteinylglycine and β-mercaptoethanol.

6. The method as claimed in claim 5, wherein the low molecular mono-thiol compound is used in an amount of about 0.1 to about 4 mmol/L.

7. The method of claim 1 where the low molecular mono-thiol compound is used at a concentration which does not significantly reduce the disulfide bonds of the irradiated protein.

8. The method as claimed in claim 1, wherein the protein retains at least 95% of its biological activity after treatment with irradiation.

9. The method as claimed in claim 7, wherein the disulfide bond reduction is detected in an screening assay using fibrinogen and SDS-PAGE (non-reducing) with coomassie blue staining which indicates the generation of free alpha-, beta- or gamma chains from fibrinogen.

10. The method as claimed in claim 9 where less than 5 % alpha, beta and gamma chains of the fibrinogen monomer are detected in the screening assay.

11. The method as claimed in claim 9 where less than 1 % alpha, beta and gamma chains of the fibrinogen monomer are detected in the screening assay.

12. The method as claimed in claim 1, wherein not more than 5% of aggregates are formed during irradiation.

13. The method as claimed in claim 1, wherein not more than 1% of aggregates are formed during irradiation.

14. The method of any of claims 1 to 13, wherein said protein containing biological composition contains purified plasma proteins.

15. The method of claim 14, wherein said plasma protein is a coagulation factor.

16. The method of claim 14, wherein said coagulation factor is selected from the group consisting of factor II, V, VII, VIII, IX, X, XI, XII and fibrinogen.

17. The method of claims 14, wherein the coagulation factor is the von Willebrand factor alone or the von Willebrand factor associated with factor VIII.

18. The method of claim 1, wherein either before, after or at the same time as said protein containing biological composition is subjected to said irradiation in the presence of any of the compounds as mentioned in claim 5, the composition is subjected to at least one different virucidal method.

19. The method of claim 18, wherein the different virucidal method is selected from the group consisting of heat treatment, pH manipulation, solvent- detergent treatment, precipitation by ammonium sulphate or polyethyleneglycol, chromatographic purification, nanofiltration and γ-irradiation treatment.

20. Use of a mono-thiol compound selected from the group consisting of cysteine, homocysteine, N-acetyl-cysteine, cysteinylglycine and β-mercaptoethanol in a virus-inactivation process.

21. A protein containing biological composition prepared by any of the methods as claimed in claims 1 to 20.
